(19) Europäisches Patentamt
European Patent Office
Office européen des brevets

(11) EP 2 114 485 B1

(12) # EUROPÄISCHE PATENTSCHRIFT

(45) Veröffentlichungstag und Bekanntmachung des Hinweises auf die Patenterteilung:
**25.03.2015 Patentblatt 2015/13**

(21) Anmeldenummer: **08707104.9**

(22) Anmeldetag: **17.01.2008**

(51) Int Cl.:
***A61M 1/16*** *(2006.01)*

(86) Internationale Anmeldenummer:
**PCT/EP2008/000339**

(87) Internationale Veröffentlichungsnummer:
**WO 2008/089913 (31.07.2008 Gazette 2008/31)**

(54) **DIALYSEMASCHINE UND VERFAHREN ZUR FESTSTELLUNG DER VERKALKUNG EINER DIALYSEMASCHINE**

DIALYSIS MACHINE AND METHOD FOR DETERMINING THE FURRING IN A DIALYSIS MACHINE

MACHINE À DIALYSE ET PROCÉDÉ DE DÉTERMINATION DE L'ENTARTRAGE D'UNE MACHINE À DIALYSE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **26.01.2007 DE 102007004115**

(43) Veröffentlichungstag der Anmeldung:
**11.11.2009 Patentblatt 2009/46**

(73) Patentinhaber: **Fresenius Medical Care Deutschland GmbH
61352 Bad Homburg (DE)**

(72) Erfinder:
- **MAIERHOFER, Andreas
97422 Schweinfurt (DE)**
- **GAGEL, Alfred
96123 Litzendorf (DE)**
- **GROSS, Malte
97464 Niederwerrn (DE)**
- **KOCH, Michael
97447 Gerolzhofen (DE)**

(74) Vertreter: **Herrmann, Uwe
Lorenz - Seidler - Gossel
Widenmayerstrasse 23
80538 München (DE)**

(56) Entgegenhaltungen:
**EP-A- 0 437 274 EP-A- 0 834 328
GB-A- 1 484 642**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 114 485 B1

## Beschreibung

Die vorliegende Erfindung betrifft eine Dialysemaschine mit wenigstens einem Filter zur Filtration der Dialysierflüssigkeit sowie mit Mitteln zur Feststellung der Verkalkung der Dialysemaschine. Die Erfindung betrifft ferner ein Verfahren zur Feststellung der Verkalkung einer Dialysemaschine.

Bei der Verwendung von Dialysierflüssigkeiten, die gleichzeitig hohe Konzentrationen an Calciumionen ($Ca^{2+}$) und Bicarbonationen ($HCO_3^-$) enthalten, kann es zur Ausfällung von Kalk ($CaCO_3$) und als Folge davon zu Kalkablagerungen in der Dialysemaschine kommen. Unter extremen Bedingungen, wie beispielsweise bei zusätzlich niedrigem Säuregehalt, bei langen Dialysezeiten oder für den Fall, dass zwischen zwei Dialysebehandlungen keine entkalkende Desinfektion durchgeführt wird, können die genannten Kalkablagerungen zum Versagen der Produktion von Dialysierflüssigkeit beispielsweise durch Blockierung der Förderpumpen oder durch Verkalkung von Dialysierflüssigkeitsfiltern führen.

Aus der EP 0 834 328 A1 ist eine Dialysemaschine bekannt, die Mittel aufweist, über die bei Feststellung eines vorbestimmten Verkalkungsgrades der Dialysemaschine eine automatische Entkalkung des Dialysierflüssigkeitkreislaufes der Maschine einleitbar ist. Dabei kann vorgesehen sein, dass der Verkalkungsgrad der Dialysemaschine durch Trübung eines Fensters eines Blutleckdetektors oder durch die Bestimmung charakteristischer Parameter der Förderpumpe für die Dialysierflüssigkeit bestimmbar ist. Bei Vorliegen von Kriterien für eine Entkalkung kann weiterhin vorgesehen sein, dass die Dialysemaschine vollautomatisch einen Entkalkungszyklus einleitet.

Während das maschinenseitige Problem der Verkalkung, das einen Abbruch der Dialyse und damit ein Nichterreichen des Behandlungsziels zur Folge haben kann, somit allgemein bekannt ist, wurden die Auswirkungen der Kalkablagerung in der Maschine auf die Calciumzufuhr auf den Patienten bisher nur wenig betrachtet. Es ist offensichtlich, dass das in der Dialysemaschine abgelagerte Calcium, das in Form von Kalk ausgefallen ist, nicht mehr den Patienten erreichen kann. In Labormessungen konnte gezeigt werden, dass die Ausfällung von $CaCO_3$ den $Ca^{2+}$-Ionengehalt am dialysatseitigen Eingang des Dialysators auf weniger als 50% der Rezeptur absenken kann. Dieses Absinken findet unter bestimmten Bedingungen deutlich schneller statt als der Ausfall der Maschine durch die Verkalkung.

Es kann somit der Fall eintreten, dass der Patient über längere Zeit mit einer Dialysierflüssigkeit behandelt wird, deren $Ca^{2+}$-Ionenkonzentration unter der Konzentration des ionisierten Calcium im Plasma liegt, was zur Folge haben kann, dass dem Patienten bei der Dialysebehandlung ungewollt Calcium entzogen wird. Das ionisierte Ca ist von hoher Bedeutung bezüglich Nervenleitung, Muskel- und Myokardkontraktilität und Blutdruck. Daher ist es vorstellbar, dass es als Folge der Maschinenverkalkung zu negativen Auswirkungen auf den Patienten kommt.

Der vorliegenden Erfindung liegt die Aufgabe zugrunde, eine Dialysemaschine der eingangsgenannten Art dahingehend weiterzubilden, dass die Verkalkung der Dialysemaschine zuverlässig erkannt werden kann, bevor die Verkalkung zum Abbruch der Behandlung führt und bevor der Patient längere Zeit mit zu geringen CalciumIonenkonzentrationen behandelt wird.

Diese Aufgabe wird durch eine Dialysemaschine mit den Merkmalen des Anspruchs 1 gelöst. Danach ist vorgesehen, dass die Maschine Mittel zur Feststellung der Verkalkung der Dialysemaschine aufweist, die einen oder mehrere Sensoren umfassen, wobei der oder die Sensoren derart ausgeführt und angeordnet sind, dass mit diesen die Ionenkonzentration oder ein für die Ionenkonzentration oder deren Änderung repräsentativer Parameter der Dialysierflüssigkeit, einer zur Entkalkung dienenden Lösung oder einer sonstigen Meßlösung entweder stromabwärts oder stromaufwärts und stromabwärts des Filters erfaßbar ist. Die Mittel zur Feststellung der Verkalkung der Dialysemaschine umfassen ferner eine Auswerte- oder Recheneinheit, die derart konfiguriert ist, dass sie auf der Grundlage der oder des mittels des oder der Sensoren erfaßten Ionenkonzentration bzw. Parameterwertes die Verkalkung der Dialysemaschine feststellt.

Bei dem oder den Sensoren kann es sich beispielsweise um Leitfähigkeitssensoren, ionenselektive Elektroden, pH-Elektroden oder um nach spektroskopischen Verfahren arbeitende Sensoren handeln.

Der Erfindung liegt somit der Gedanke zugrunde, dass stromabwärts oder auch stromaufwärts und stromabwärts des oder der Filter die Ionenkonzentration, vorzugsweise die $Ca^{2+}$-Ionenkonzentration, oder ein für die Ionenkonzentration oder deren Änderung repräsentativer Parameter, wie beispielsweise die Leitfähigkeit, ermittelt wird und auf dieser Grundlage auf die Verkalkung des Filters rückgeschlossen werden kann. Dabei kann die Ionenkonzentration oder der genannte Parameter der Dialysierflüssigkeit, einer zur Entkalkung vorgesehenen Lösung oder einer sonstigen Lösung erfasst werden. Diese sonstige Lösung kann eine ionenhaltige Lösung sein, die beispielsweise $Ca^{2+}$ oder $H^+$-Ionen enthält. Dabei kann vorgesehen sein, dass wenigstens einem der Filter zwei Sensoren zugeordnet sind, von denen einer stromaufwärts und einer stromabwärts des Filters angeordnet ist. Denkbar ist, die Überwachung des Filters bzw. die Ermittlung des Verkalkungsgrades des Filters durch Vergleich zweier Leitfähigkeitsmeßwerte vorzunehmen, von denen einer unmittelbar vor und einer nach dem zu überprüfenden Filter aufgenommen wird. Eine Verkalkung des Filters liegt dann vor, wenn der stromaufwärts des Filters gemessene Leitfähigkeitswert größer ist als der stromabwärts des Filters gemessene Leitfähigkeitswert.

Der Begriff der Erfassung der "Ionenkonzentration oder eines für die Ionenkonzentration oder deren Änderung

repräsentativen Parameters" ist weit auszulegen und umfaßt unter anderem auch indirekte Meßmethoden zur Ionenmessung. Denkbar und von der Erfindung umfaßt ist beispielsweise die Messung von $CO_2$, das bei der Entkalkung entsteht. Die $CO_2$-Konzentration steht über das chemische Gleichgewicht mit der Konzentration anderer Ionen in Zusammenhang und bildet somit ein Maß für die Ionenkonzentration, so dass die Messung beispielsweise der $CO_2$-Menge, der $CO_2$-Konzentration bzw. des $CO_2$-Volumens eine Ausführungsform der Erfassung eines für die Ionenkonzentration repräsentativen Parameters darstellt.

Denkbar ist, dass wenigstens einem der Filter sowohl auf der Primärseite als auch auf der Sekundärseite des Filters stromaufwärts und stromabwärts jeweils ein Sensor zugeordnet ist. Diese Anordnung ermöglicht es, sowohl die primärseitige als auch sekundärseitige Verkalkung des Filters zu erfassen.

In weiterer Ausgestaltung der Erfindung ist vorgesehen, dass wenigstens eine absperrbare Bypassleitung angeordnet ist, die im geöffneten Zustand eine Strömungsverbindung zweier Sensoren unter Umgehung wenigstens eines der Filter herstellt. Soll der Einfluß des Filters auf die mittels der Sensoren erhaltenen Meßwerte ausgeschlossen werden, werden diese unter Umgehung des Filters derart miteinander verbunden, dass die Dialysierflüssigkeit, die zur Entkalkung dienende Lösung oder eine Meßlösung erst den einen und dann den anderen Sensor durchströmt. Dies ermöglicht es, die Meßwerte der Sensoren miteinander vergleichen zu können bzw. eine Kalibrierung der Sensoren vornehmen zu können.

In weiterer Ausgestaltung der Erfindung ist vorgesehen, dass pro Filter oder für mehrere Filter nur ein Sensor vorgesehen ist, der stromabwärts des oder der Filter angeordnet ist. Eine Überwachung der Verkalkung des Filters ist somit auch nur mit einem Sensor, beispielsweise mit einer Leitfähigkeitsmeßzelle möglich, die sich stromabwärts des Filters befindet. Eine Bestimmung der Verkalkung kann nun z. B. dadurch erfolgen, dass vor Beginn oder zu Beginn der Behandlung der Leitfähigkeitsmeßwert ermittelt wird und während der Behandlung oder auch nach der Behandlung geprüft wird ob und inwieweit eine Messwertveränderung eingetreten ist.

Besonders vorteilhaft ist es, wenn wenigstens eine absperrbare Bypassleitung vorgesehen ist, die derart angeordnet ist, dass sie im geöffneten Zustand die Dialysierflüssigkeit, die zur Entkalkung dienende Lösung oder die sonstige Meßlösung unter Umgehung wenigstens eines Filters dem Sensor zuführt. Somit ist es möglich, auch nur mit einem Sensor, beispielsweise mit einer stromabwärts des Filters angeordneten Leitfähigkeitsmeßzelle die Ionenkonzentration bzw. den für diese oder deren Änderung repräsentativen Parameter stromaufwärts und stromabwärts des Filters zu ermitteln. Soll die Ionenkonzentration bzw. der genannte Parameter stromaufwärts des Filters erfaßt werden, wird die Bypassleitung geöffnet und dem Sensor die stromaufwärts des Filters mittels der Bypassleitung abgezogene Lösung zugeführt. Für die Erfassung des Meßwertes bei Durchströmung des Filters wird die Bypassleitung geschlossen und dementsprechend der Filter durchströmt. Ein Vorteil dieses Verfahrens ist es, dass nur ein Sensor pro Filter oder auch nur ein Sensor für mehrere Filter mit dazugehöriger Elektronik benötigt wird. Zudem entfällt ein Abgleich von mehreren Sensoren relativ zueinander.

Die Erfindung betrifft des weiteren ein Verfahren zur Feststellung der Verkalkung einer Dialysemaschine, insbesondere einer Dialysemaschine gemäß einem der Ansprüche 1 bis 8, wobei die Dialysemaschine wenigstens einen Filter zur Filtration der Dialysierflüssigkeit aufweist.

Das Verfahren ist dadurch gekennzeichnet, dass zur Bestimmung der Verkalkung der Dialysemaschine die Ionenkonzentration oder ein für die Ionenkonzentration oder deren Änderung repräsentativer Parameter der Dialysierflüssigkeit oder einer zur Entkalkung dienenden Lösung oder einer Meßlösung entweder stromabwärts oder stromaufwärts und stromabwärts des Filters gemessen wird und dass auf der Grundlage des oder der gemessenen Ionenkonzentration bzw. Parameterwertes die Verkalkung festgestellt wird. Wie oben ausgeführt, kann es sich bei dem für die Ionenkonzentration repräsentativen Parameter beispielsweise um die Leitfähigkeit, den pH-Wert oder einen mittels einer ionenselektiven Elektrode oder mittels spektroskopischer Verfahren ermittelten Parameter, wie beispielsweise die Absorption oder Transmission handeln. Vorzugsweise handelt es sich bei der Ionenkonzentration um die $Ca^{2+}$-Ionenkonzentration oder um die $H^+$-Ionenkonzentration.

Dem wenigstens einem Filter können zwei Sensoren zugeordnet sein, die stromaufwärts und stromabwärts des Filters angeordnet sind. Der Grad der Verkalkung kann durch einen Vergleich der stromaufwärts und stromabwärts gemessenen Ionenkonzentration bzw. des für diese oder deren Änderung repräsentativen Parameters ermittelt werden. Dabei kann vorgesehen sein, dass zu einem ersten Zeitpunkt, vorzugsweise vor oder zu Beginn einer Behandlung, Differenzen in den Meßwerten der Sensoren ermittelt werden, und diese Differenzen sodann zu einem zweiten, nach dem ersten Zeitpunkt liegenden Zeitpunkt, insbesondere während der Behandlung, bei der Ermittlung des Grades der Verkalkung berücksichtigt werden. Denkbar ist auch, dass die genannten Meßwertdifferenzen durch entsprechende Kalibrierung der Sensoren beseitigt werden.

In weiterer Ausgestaltung der Erfindung ist vorgesehen, dass wenigstens eine absperrbare Bypassleitung vorgesehen ist, die im geöffneten Zustand eine Verbindung zweier Sensoren unter Umgehung wenigstens eines der Filter herstellt und dass zum Zwecke der Ermittlung von Meßwertdifferenzen oder zur Kalibrierung der Sensoren die Bypassleitung geöffnet wird.

Pro Filter oder für mehrere Filter kann auch nur ein Sensor vorgesehen sein, der stromabwärts des oder der

Filter angeordnet ist, wobei zu einem ersten Zeitpunkt, vorzugsweise vor oder zu Beginn einer Behandlung, mittels des Sensors ein Meßwert ermittelt wird und dass zu einem zweiten, nach dem ersten Zeitpunkt liegenden Zeitpunkt, insbesondere während der Behandlung, überprüft wird, ob eine Meßwertänderung eingetreten ist.

Auch ist es denkbar, dass pro Filter oder für mehrere Filter nur ein Sensor vorgesehen ist, der stromabwärts des oder der Filter angeordnet ist und dass wenigstens eine absperrbare Bypassleitung vorgesehen ist, die derart angeordnet ist, dass sie im geöffneten Zustand die Dialysierflüssigkeit, die zur Entkalkung dienende Lösung oder die sonstige Meßlösung unter Umgehung wenigstens eines Filters dem Sensor zuführt, wobei zu Beginn oder während der Behandlung der Meßwert des Sensors bei geöffneter Bypassleitung ermittelt wird und dieser Meßwert mit dem Meßwert verglichen wird, der nach der Durchströmung des Filters erhalten wird.

In weiterer Ausgestaltung der Erfindung ist vorgesehen, dass eine Entkalkung vorgenommen wird und dass nach oder während der Entkalkung eine Bewertung der Entkalkung vorgenommen wird, die auf einem Vergleich von Meßwerten des einen oder der mehreren Sensoren basiert, mittels derer die Ionenkonzentration oder der für die Ionenkonzentration oder deren Änderung repräsentative Parameter stromaufwärts und stromabwärts des Filters erfaßt wird.

Weiterhin kann vorgesehen sein, dass nach der Entkalkung eine Bewertung der Entkalkung vorgenommen wird, die auf einem Vergleich von Meßwerten eines Sensors vor und nach der Entkalkung basiert, wobei der Sensor stromabwärts des oder der Filter angeordnet und derart ausgeführt ist, dass mit diesem die Ionenkonzentration oder der für diese oder deren Änderung repräsentative Parameter erfaßt wird.

Bei der Ionenkonzentration kann es sich beispielsweise um die $Ca^{2+}$-Ionenkonzentration oder auch um die $H^{+}$-Ionenkonzentration handeln.

Weitere Einzelheiten und Vorteile der Erfindung werden anhand eines in der Zeichnung dargestellten Ausführungsbeispiels näher erläutert. Es zeigen:

- Figur 1: zeitlicher Verlauf der Leitfähigkeit bei Ca-Ausfällung bei stehender Dialysierflüssigkeit mit Kontakt zur Atmosphäre,
- Figur 2: Flußplan eines Dialysegerätes mit Onlineaufbereitung der Dialysierflüssigkeit,
- Figur 3: zeitlicher Verlauf der $Ca^{2+}$-Ionenkonzentration sowie der Leitfähigkeit während einer Dialysebehandlung mit frischer Dialysierflüssigkeit vor und nach Entkalkung,
- Figur 4: zeitlicher Verlauf der $Ca^{2+}$-Ionenkonzentration sowie der Leitfähigkeit beim Ein- und Ausbau von verkalkten Filtern,
- Figur 5: schematische Darstellung der Verkalkungsüberwachung mit zwei Leitfähigkeitsmeßzellen pro Filter,
- Figur 6: schematische Darstellung der Verkalkungsüberwachung mit zwei Leitfähigkeitsmeßzellen pro Filter und Bypass-Schaltung,
- Figur 7: schematische Darstellung der Verkalkungsüberwachung mit nur einer Leitfähigkeitsmeßzelle pro Filter und Bypass-Schaltung,
- Figur 8: Flußplan des Dialysegerätes gemäß Figur 2 mit Verkalkungsüberwachung aller Filter durch zusätzliche Leitfähigkeitsmeßzellen,
- Figur 9: Flußplan des Dialysegerätes gemäß Figur 2 mit Verkalkungsüberwachung aller Filter durch zusätzliche Leitfähigkeitsmeßzellen mit Kalibriermöglichkeit und
- Figur 10: Flußplan des Dialysegerätes gemäß Figur 2 mit Verkalkungsüberwachung aller Filter durch eine Leitfähigkeitsmeßzelle und Bypass-Schaltungen.

Die Leitfähigkeit der Dialysierflüssigkeit wird durch ihre einzelnen Komponenten bestimmt. Der Hauptbeitrag der Leitfähigkeit ist auf NaCl zurückzuführen, jedoch auch die in relativ geringen Konzentrationen vorhandenen $Ca^{2+}$-Ionen tragen zur Leitfähigkeit bei. Steht eine sowohl $Ca^{2+}$ als auch $HCO_3^-$ enthaltende Dialysierflüssigkeit in Kontakt mit der Atmosphäre, so führt das Ausgasen von $CO_2$ zu einem Verbrauch von Hydrogencarbonat und somit zu einer pH-Wertverschiebung ins Alkalische, die wiederum eine Ausfällung von Calciumkarbonat bewirkt. Dies wird durch die nachstehende Reaktionsgleichung verdeutlicht:

$$Ca^{2+} + 2HCO_3^- \rightarrow CaCO_3 \downarrow + CO_2 \uparrow + H_2O$$

Hiermit werden der Dialysierflüssigkeit Ionen entzogen, so dass die Leitfähigkeit der Lösung sinkt. Dies konnte in Laborversuchen nachgewiesen werden. Figur 1 zeigt den Abfall der Leitfähigkeit über der Zeit bei stehender Dialysierflüssigkeit mit Kontakt zur Atmosphäre. Wie dies deutlich aus Figur 1 ersichtlich ist, nimmt die Leitfähigkeit der Lösung mit der Zeit ab. Wie dies aus den in Figur 1 angegebenen Meßwerten weiter ersichtlich ist, ist dies im wesentlichen auf die Verringerung des Gehalts an Calciumionen sowie parallel dazu auf den Verbrauch von Hydrogencarbonationen zurückzuführen.

Über die Änderung der Leitfähigkeit ist im Prinzip auch eine Quantifizierung der Änderung der $Ca^{2+}$-Ionenkonzentrationen in der Dialysierflüssigkeit möglich:

Empirische Untersuchen des Einflusses der $Ca^{2+}$-Ionenkonzentration auf die Leitfähigkeit in typischen Dialysierflüssigkeiten ergaben folgenden Zusammenhang:

$$\Delta c = \left(110 \frac{\mu S / cm}{mmol / l}\right)^{-1} \Delta LF$$

Bei einer Meßgenauigkeit für die Leitfähigkeit von 10 µS/cm können somit Änderungen in der $Ca^{2+}$-Ionenkonzentration durch Ausfällung von $CaCO_3$ mit einer Genauigkeit von 0,1 mol/l quantifiziert werden.

Wird dagegen die Dialysierflüssigkeit mit einem Dialysegerät kontinuierlich frisch durch Mischung von RO-Wasser mit einer sauren und einer bicarbonathaltigen Komponente erzeugt, so ist innerhalb der kurzen Zeit zwischen der Erzeugung und dem Transport zum Dialysator zunächst keine Ausfällung zu erwarten.

Zur weiteren Verdeutlichung der Problematik des Ausfalls von $CaCO_3$ wird zunächst auf Figur 2 verwiesen. Diese Figur zeigt einen Flußplan eines Dialysegerätes mit Online-Aufbereitung des Dialysats aus RO-Wasser, Säure und Bicarbonat. Wie dies aus Figur 2 hervorgeht, sind stromaufwärts des Dialysators D3 zwei Sterilfilter D1 und D2 angeordnet. Dabei sind die Sterilfilter D1 und D2 derart angeordnet, dass die zu dem Dialysator D3 gelangende Dialysierflüssigkeit von der Primärzu der Sekundärseite des ersten Sterilfilters D1 gelangt und sodann die Primärseite des zweiten Sterilfilters D2 durchströmt. In diesem wird Substituat gewonnen, das dem Blut des Patienten im Bedarfsfall zugegeben wird, wie dies bei der Hämofiltration und Hämodiafiltration der Fall ist. Die vorliegende Erfindung ist selbstverständlich nicht auf die Hämodiafiltration oder Hämofiltration beschränkt, sondern umfasst u. a. auch das Verfahren der Hämodialyse.

Wie dies weiter aus Figur 2 hervorgeht, ist stromaufwärts des zweiten Sterilfilters D2 eine Leitfähigkeitsmeßzelle 20 und stromabwärts des Dialysators D3 sowie des ersten Sterilfilters D1 eine zweite Leitfähigkeitsmeßzelle 40 angeordnet.

Die Leitfähigkeit der Dialysierflüssigkeit wurde mittels der Leitfähigkeit der Meßzelle 20 kontinuierlich gemessen.

Zusätzlich wurden in unmittelbar stromaufwärts des Dialysators D3 gewonnen Proben der Dialysierflüssigkeit die $Na^+$- und $Ca^{2+}$-Ionenkonzentration bestimmt.

In Figur 3 sind die Leitfähigkeitsmeßwerte (durchgezogene Linie) und die Meßwerte der Ca-Ionenkonzentration (Kreise) angegeben. Bei einem Dialysat mit hohem $Ca^{2+}$- und $HCO_3^-$-Gehalt ergab sich nach ca. 2 h ein deutlicher Abfall der $Ca^{2+}$-Ionenkonzentration sowie der Leitfähigkeit der Dialyselösung. Nach intensivem Spülen mit einem entkalkenden Reinigungsmittel, beispielsweise Peressigsäure, wurden wieder die ursprünglichen $Ca^{2+}$-Ionenkonzentrationen gemessen, wie dies aus Figur 3 hervorgeht. Die Leitfähigkeit stieg ebenfalls wieder an, wobei jedoch Effekte durch Zellendrifts über die Zeit sowie ein potentieller Effekt der Verkalkung der Leitfähigkeitsmeßzelle selbst zu berücksichtigen sind. Die $Na^+$-Ionenkonzentration blieb in allen Fällen konstant, so dass Änderungen im Mischungsverhältnis bei der Erzeugung der Dialysierflüssigkeit ausgeschlossen werden können.

Das in Figur 3 dargestellte Verhalten des Abfallens des Leitfähigkeit sowie der Calcium-Ionenkonzentration kann dadurch erklärt werden, dass bei hoher $Ca^{2+}$-Ionenkonzentration sowie $HCO_3^-$-Ionenkonzentrationen die Dialysierflüssigkeit übersättigt ist, so dass das Vorliegen von Kristallisationskeimen, rauhen Oberflächen oder Druckschwankungen durch das Fördern der Dialysierflüssigkeit mittels Förderpumpe zur spontanen Ausfällung von $CaCO_3$ führt.

In Laborversuchen konnte nun des weiteren gezeigt werden, dass insbesondere die Dialysierflüssigkeitsfilter D1 und D2 nicht nur die bevorzugten Orte der Verkalkung sind, sondern bei beginnender Verkalkung selbst die weitere Ausfällung fördern:

Um dies zu verifizieren, wurden an einem Dialysegerät im laufenden Betrieb die Dialysierflüssigkeitsfilter D1 und

D2 gemäß Figur 2 ein- und ausgebaut. Die Leitfähigkeit wurde mittels der Leitfähigkeitsmeßzellen 20 und 40 kontinuierlich gemessen. Wie auch oben beschrieben, wurden zusätzlich in stromaufwärts des Dialysators D3 gewonnenen Dialysatproben die Natrium- und Calcium- Ionenkonzentrationen bestimmt.

Wie dies aus Figur 4 hervorgeht, sank die Leitfähigkeit bei Einsetzen des Filters D1 sowohl an den Sensoren 20 (LF2) als auch 40 (LF4) um ca. 60 $\mu$S/cm gegenüber der Leitfähigkeit ohne Filter. Zugleich mit der Leitfähigkeitsänderung konnte ein Absinken der $Ca^{2+}$-Ionenkonzentration vor dem Dialysator D3 bei Einsetzen des Filters D1 um 0,6 mmol/l ermittelt werden.

Wurde zusätzlich ein verkalkter Filter D2 eingebaut, so sank die Leitfähigkeit (LF4) am Sensor 40 um weitere ca. 50 $\mu$S/cm und die $Ca^{2+}$-Ionenkonzentration um weitere 0,4 mmol/l, da die Flußstrecke zu dem Sensor 40 nunmehr zwei verkalkte Filter D1, D2 enthielt. Die Leitfähigkeit (LF2) an der Leitfähigkeitsmeßzelle 20 blieb konstant.

Nach Entfernen der beiden Filter D1, D2 erreichte die Leitfähigkeit und die $Ca^{2+}$-Ionenkonzentration an beiden Meßzellen wieder den Ursprungswert, wie dies aus Figur 4 hervorgeht.

Zur Klarstellung sei an dieser Stelle darauf hingewiesen, dass der Kurvenverlauf der Größe "LF4" in Figur 4 nicht etwa nur in dem Bereich "mit Filtern D1 und D2", sondern auch in dem davor liegenden Zeitraum existiert, in dem er sich von der Kurve der Größe "LF2" nicht deutlich abhebt.

Ferner wird darauf hingewiesen, dass es sich bei den Messkurven gemäß der Figur 3 und Figur 4 um Messungen handelt, die mit einem in-vitro Aufbau erhalten wurden, wobei statt Blut eine Vergleichsflüssigkeit im extrakorporalen Blutkreislauf zirkuliert wurde. Für das Entstehen von Verkalkung existieren günstige und ungünstige Zusammensetzungen für die Dialysierflüssigkeit, insbesondere bezüglich ihres Bicarbonat-, Kalzium- und Acetatgehaltes. Für die Versuche wurden nun solche Kombinationen gewählt, bei denen die Verkalkung eher schnell als langsam voranschreitet. Die gezeigten Daten und Kurven sind daher für eine durchschnittliche Dialysebehandlung nicht repräsentativ. Es handelt sich bei den dargestellten Messungen vielmehr um extreme Situationen, mittels derer die im Rahmen dieser Erfindung eine Rolle spielenden Effekte besser verdeutlicht werden können.

Die Werte der $Ca^{2+}$-Ionenkonzentration befinden sich in guter Übereinstimmung mit der nach obiger Gleichung aus der Leitfähigkeitsänderung berechneten Änderung der $Ca^{2+}$-Ionenkonzentration. Die $Na^{+}$-Ionenkonzentration blieb in allen Fällen konstant, so dass Änderungen im Mischungsverhältnis bei der Erzeugung der Dialysierflüssigkeit ausgeschlossen werden können.

Die Erkennung der Verkalkung beruht in dem hier dargestellten Ausführungsbeispiel auf einer Änderung der Leitfähigkeit bei konstant bleibendem Mischungsverhältnis bei der Erzeugung der Dialysierflüssigkeit, wobei die Änderung der Leitfähigkeit durch die Ausfällung des Calciums in Form von Calciumkarbonat bedingt ist. Daher muss sichergestellt sein, dass über die Dauer der Vergleichsmessungen sowohl das Dosiersystem als auch die Leitfähigkeitsmessung keinen Drifts unterliegen.

Die Laborexperimente wurden nur mit Zusammensetzungen der Dialysierflüssigkeit durchgeführt, die zu einer Übersättigung mit $CaCO_3$ führten.

In einer Ausgestaltung der Erfindung besteht eine Vorrichtung zur Erkennung der Verkalkung der Dialysierflüssigkeitsfilter im Dialysegerät oder des Dialysators aus zwei Leitfähigkeitsmeßzellen, die derart angeordnet sind, dass die Leitfähigkeit der Dialysierflüssigkeit oder auch einer zur Entkalkung dienenden Lösung oder einer sonstigen Meßlösung vor und nach dem Durchtritt durch den Filter bzw. den Dialysator gemessen wird. Eine solche Ausgestaltung der Erfindung ist in Figur 5 dargestellt, wobei die Leitfähigkeitsmeßzellen mit den Bezugszeichen 1, 2 und der Filter mit dem Bezugszeichen D angegeben ist.

Die Leitfähigkeitsmeßzellen 1, 2 müssen zuvor aufeinander kalibriert werden. Soll der Einfluss des Filters D dabei sicher ausgeschlossen werden, ist ein Bypass 100 vorzusehen, der den Filter D umgibt, wie dies aus Figur 6 hervorgeht. Die Bypassleitung 100 ist durch ein Ventil absperrbar. Ebenso ist die zum Filter D führende Leitung durch ein Ventil absperrbar. Dementsprechend kann je nach Schaltung der in Figur 6 dargestellten Ventile entweder der Filter D oder die Bypassleitung 100 durchströmt werden. Soll ein Abgleich der Leitfähigkeitsmeßzellen 1, 2 durchgeführt werden, wird die Bypassleitung 100 durchströmt, soll die Verkalkung des Filters D ermittelt werden, wird die Bypassleitung 100 geschlossen, der Filter durchströmt und mittels der Leitfähigkeitsmeßzellen 1, 2 ermittelt, ob ein Unterschied in den stromaufwärts und stromabwärts des Filters D ermittelten Leitfähigkeitswerten festgestellt werden kann.

Auch ist es alternativ dazu möglich, die Überwachung der Verkalkung durch nur eine Leitfähigkeitsmeßzelle durchzuführen, die in Figur 7 mit dem Bezugszeichen 2 dargestellt ist. Diese Meßzelle befindet sich stromabwärts des Filters D. Des weiteren ist eine Bypassleitung 200 vorgesehen, die den Filter D umgibt. Wie dies aus Figur 7 hervorgeht, ist die Bypassleitung 200 ebenfalls durch ein Ventil absperrbar. Dasselbe gilt für die zum Filter D führende Leitung. Soll die Ionenkonzentration bzw. ein für diese repräsentativer Parameter stromaufwärts des Filters D ermittelt werden, wird die Bypassleitung 200 geöffnet und der Leitfähigkeitswert mittels der Leitfähigkeitsmeßzelle 2 erfasst. Soll die Leitfähigkeit nach Durchströmung des Filters D erfasst werden, wird die zum Filter D führende Leitung geöffnet und die Bypassleitung 200 geschlossen.

Die Anordnung gemäß Figur 7 bietet den Vorteil, dass nur eine Leitfähigkeitsmeßzelle mit dazugehöriger

Elektronik benötigt wird und zudem ein Abgleich von mehreren Meßzellen auch entfallen kann. Als Nachteil ist zu nennen, dass hier bei Umschaltung der Flusswege zur Messung der Leitfähigkeit Stabilisierungszeiten anfallen. Des weiteren fallen längere Zeiten an, in denen gegebenenfalls keine Dialyse möglich ist, wenn für die Behandlung des Patienten die Durchströmung des Filters D erforderlich ist.

Figur 8 zeigt das Flußschema gemäß Figur 2 mit einer Anordnung von Leitfähigkeitsmeßzellen nach dem in Figur 5 beschriebenen Prinzip. Zur Verkalkungsüberwachung des Sterilfilters D1 dienen die Leitfähigkeitsmeßzellen 10, 20 bzw. für die Primärseite des Sterilfilters D1 die Meßzellen 10, 40. Zur Überwachung des Sterilfilters D2 dienen die Leitfähigkeitsmeßzellen 20, 30 und zu der des Dialysators D3 die Meßzellen 30, 40.

Bei gleichzeitiger Kalibriermöglichkeit nach dem in Figur 6 beschriebenen Prinzip ergibt sich ein Flußplan gemäß Figur 9. Wie dies aus Figur 9 hervorgeht, sind jeweils Bypassleitungen 100 vorgesehen, die derart angeordnet sind, dass jede der Leitfähigkeitsmeßzellen 10, 20, 30 in Reihe mit der Leitfähigkeitsmeßzelle 40 geschaltet werden kann. Dementsprechend erfolgt der Abgleich der Leitfähigkeitsmeßzellen hier durch entsprechende Ventilschaltungen, indem nacheinander die Leitfähigkeitsmeßzellen 10, 20 und 30 unter Umgehung des jeweils nachgelagerten Filters D1, D2, D3 in Reihe mit der Leitfähigkeitsmeßzelle 40 geschaltet werden. Dieser Abgleich ist vor Beginn der Dialyse möglich. Während der Dialyse werden die Ventile dann derart geschaltet, dass sich der Flußplan nach Figur 8 ergibt.

Wie dies des weiteren aus Figur 10 hervorgeht, ist durch das Einführen von Bypassleitungen 200 eine Überwachung aller Filter D1, D2, D3 auch mit nur einer Leitfähigkeitsmeßzelle 40 erreichbar.

Die Leitfähigkeitsmeßzelle 40 ist stromabwärts der Filter D1, D2 sowie des Dialysators D3 angeordnet. Für eine Referenzmessung werden die Bypassleitungen 200 mit den Ventilen V3, V6 und V7 geöffnet und die Ventile V2, V5 und V8 in den Zuleitungen zu den Filtern D1, D2 sowie des Dialysators D3 geschlossen, so dass die Dialysierflüssigkeit im Bypass um die Filter D1, D2 und den Dialysator D3 strömt. Die Ventile V10 und V9 sind ebenfalls geschlossen.

Soll der Filter D1 auf Verkalkung überprüft werden, werden die Ventile V2, V6 und V7, geöffnet, so dass die Dialysierflüssigkeit nur durch den Filter D1 von der Primärauf die Sekundärseite strömt, während bei geöffneten Ventilen V6 und V7 ein Bypass um den Filter D2 und den Dialysator D3 erfolgt. Alle anderen Ventile sind in diesem Fall geschlossen.

Soll eine primärseitige Überprüfung des Sterilfilters D1 vorgenommen werden, werden die Ventile V2 und V10 geöffnet und alle anderen Ventile geschlossen. In diesem Fall durchströmt die Dialysierflüssigkeit das Ventil V2, sodann die Primärseite des Sterilfilters D1 und dann das Ventil V10, um schließlich zu der Leitfähigkeitsmeßzelle 40 zu gelangen.

Eine Überprüfung des Verkalkungszustandes des Sterilfilters D2 erfolgt durch Öffnen der Ventile V3, V5, V7 und durch Schließen aller weiterer Ventile. Eine Überprüfung des Dialysators D3 erfolgt bei geöffneten Ventilen V3, V6, V8, V9, während alle weiteren Ventile geschlossen sind.

Die im folgenden angegebene Tabelle faßt nochmals zusammen, welche Ventile zur Prüfung welches Filters bzw. des Dialysators offen und geschlossen sind und gibt gleichzeitig an, welche Leitfähigkeitsmeßzelle durch die jeweiligen Anordnungen ersetzt werden kann (Bezugszeichen von Fig. 9).

| Prüfung | Offen | Geschlossen | Ersetzte LF-Zelle |
|---|---|---|---|
| Referenz | V3,V6,V7 | V2,V5,V8,V9,V10 | LF1 (10) |
| D1 | V2,V6,V7 | V3,V5,V8,V9,V10 | LF2 (20) |
| D1, nur Primärseite | V2, V10 | V3,V5,V6,V7,V8,V9 | / |
| D2 | V3, V5, V7 | V2,V6, V8,V9,V10 | LF3 (30) |
| D3 | V3, V6, V8, V9 | V2,V5,V7,V10 | / |

Bei diesen theoretisch möglichen Schaltungsmöglichkeiten wurde noch nicht berücksichtigt, dass bestimmte Schaltungen wegen des Fehlens von für eine Dialysebehandlung vorgeschriebenen Leitfähigkeitssensoren und/oder Filterstufen für die Dialysierflüssigkeit in der Praxis weniger relevant sein können.

Gemäß dem in Figur 10 beschriebenen Prinzip ist auch eine teilweise Reduktion der Anzahl der in den Figuren 8 und 9 aufgeführten Leitfähigkeitsmeßzellen möglich.

Zur kontinuierlichen Überwachung der Maschinenverkalkung kann nun kontinuierlich die Leitfähigkeit gemessen werden. Dabei müssen eventuelle Drifts in der Leitfähigkeitsmessung kompensiert werden, um Änderungen in der Leitfähigkeit (nicht Absolutwert der Leitfähigkeit) mit einer Präzision von 0,01 mS/cm erkennen zu können.

Der Ablauf der kontinuierlichen Überwachung der Maschinenverkalkung in einer Anordnung gemäß Figur 9 gestaltet sich wie folgt:

Nach einer Kalibrierung aller Leitfähigkeitsmeßzellen 10, 20, 30, 40 wird mit diesen kontinuierlich die Leitfähigkeit gemessen. Die Überwachung der Filter D1 und D2 erfolgt durch Vergleich der jeweils unmittelbar vor bzw. nach dem jeweiligen Filter gemessenen Leitfähigkeit. Eine Verkalkung des Filters liegt dann vor, wenn die nach dem

Filter gemessene Leitfähigkeit kleiner ist als die vor dem Filter gemessene Leitfähigkeit. Mittels der oben angegebenen Gleichung kann auf den Grad der Verkalkung in Form einer Konzentrationsdifferenz geschlossen werden. Wesentlich ist, dass bei Änderungen der Dialysatzusammensetzung während der Behandlung hinreichend lange Stabilisierungszeiten abzuwarten sind.

Mit dem erfindungsgemäßen Verfahren kann eine initial bereits vorliegende Verkalkung der Filter ohne weiteres erkannt werden.

Soll der Dialysator D3 überwacht werden, ist es nötig, den Einfluss des Patienten durch ein Anhalten der Blutpumpe auszuschalten. Zugleich sollte zur schnellen Stabilisierung der Leitfähigkeit an den Leitfähigkeitsmeßzellen 30, 40 der Fluß der Dialysierflüssigkeit möglichst hoch eingestellt werden. Die Auswertung erfolgt dann wie oben beschrieben wie für die Filter D1 und D2.

Soll keine Kalibrierung der Leitfähigkeitsmeßzellen über etwaige Bypassleitungen vorgenommen werden, wie dies für Figur 8 gilt, müssen zu Beginn der Behandlung ohne Einfluss des Patienten bei stabiler Leitfähigkeit etwaige Meßdifferenzen der einzelnen Leitfähigkeitsmeßzellen 10, 20, 30 ,40 zueinander ermittelt werden. Unter Berücksichtigung dieser initialen Meßdifferenzen ist dann die Überwachung wie oben beschrieben möglich. Allerdings kann auf diese Weise eine bereits zu Beginn der Behandlung vorliegende Verkalkung nicht genau erfaßt werden, was einen Nachteil gegenüber der Anordnung gemäß Figur 9 darstellt.

Wie oben ausgeführt, ist es auch möglich, dass nicht pro Filter zwei Leitfähigkeitsmeßzellen, sondern nur eine Leitfähigkeitsmeßzelle angeordnet ist. Dabei kann die eine Leitfähigkeitsmeßzelle auch mehreren Filtern zugeordnet sein, wie dies in Figur 10 dargestellt ist. Hierbei wird für jeden zu überwachenden Filter D1, D2 eine stromabwärts dieses Filters liegende Meßzelle gemäß Figur 7 bzw. Figur 10 benötigt. Sollen keine den Filter umgehenden Bypässe vorgesehen werden, wie dies beispielsweise in Figur 7 der Fall ist, und bleibt die Zusammensetzung der Dialysierlösung während der gesamten Behandlung konstant, so wird zu Beginn der Behandlung ein Anfangswert der Leitfähigkeit $LF_0$ ermittelt und gespeichert. Ein Absinken der im weiteren Verlauf der Behandlung kontinuierlich gemessenen Leitfähigkeit gegenüber dem Wert $LF_0$ deutet dann auf eine Zunahme der Verkalkung hin.

Eine bereits zu Beginn vorliegende Verkalkung kann so nicht erkannt werden.

Ändert sich die Zusammensetzung der Dialysierflüssigkeit während der Behandlung, wird beispielsweise der Sollnatriumwert durch den Benutzer während der Behandlung verstellt, so kann mittels einer empirisch ermittelten Formel auf Grundlage der Konzentratzusammensetzung und der zu Beginn gemessenen Leitfähigkeit ein Erwartungswert für die Leitfähigkeitsänderung berechnet werden. Unterschreitet die gemessene Leitfähigkeit die erwartete Leitfähigkeit, so ist dies ein Anzeichen für die Verkalkung.

Sollen die Filter jeweils mit Bypässen ausgestaltet werden, kann der volle Umfang einer kontinuierlichen sowie initialen Verkalkungsüberwachung aller Filter wie oben beschrieben erreicht werden.

Die Ursache für das Vorliegen verkalkter Filter kann beispielsweise in einer unzureichenden Entkalkung zwischen Behandlungen liegen. Andere Ursachen sind der Einsatz bereits verkalkter Filter bzw. die Mehrfachverwendung von Dialysatoren. Wie oben ausgeführt, besteht dabei die Gefahr des Ausfalls des Dialysegerätes durch Kalkablagerungen und gegebenenfalls die Patientengefährdung durch zu niedrige $Ca^{2+}$-Ionenkonzentration der Dialysierflüssigkeit.

Wie oben ausgeführt, kann die Verkalkungserkennung darin bestehen, dass eine Konzentratmischung so eingestellt wird, dass eine übersättigte Lösung vorliegt rund dass sodann die Leitfähigkeitsmeßwerte stromaufwärts und stromabwärts des zu untersuchenden Filters und/oder Dialysators aufgenommen werden. Werden Bypass-Schaltungen eingesetzt, ist jeweils eine hinreichend lange Stabilisierungszeit einzuhalten. Ist die stromabwärts gemessene Leitfähigkeit geringer als die stromaufwärtsgemessene Leitfähigkeit, kann auf die Verkalkung geschlossen werden, wobei deren Grad über die oben angegebene Gleichung ermittelt werden kann.

Soll der Entkalkungsvorgang selbst überwacht werden, kann beispielsweise die Messung der Leitfähigkeit vor und nach Durchführung des Entkalkungszyklus bzw. auch während der Entkalkung erfolgen.

Vor Durchführung der Entkalkungsprozedur kann für den zu überwachenden Filter durch das oben beschriebene Verfahren (Messung der Leitfähigkeit stromaufwärts und stromabwärts des Filters) der Grad der Verkalkung bestimmt werden. Nach Durchführung der Entkalkung erfolgt eine erneute Bestimmung der Leitfähigkeit stromaufwärts und stromabwärts des Filters. Der Filter ist dann vollständig entkalkt, wenn die Leitfähigkeit stromaufwärts und stromabwärts des Filters identisch ist. Eine Bewertung der Entkalkung ist auch dann möglich, wenn nur eine Leitfähigkeitsmeßzelle stromabwärts des Filters angeordnet ist. Dies gilt beispielsweise für den Filter D1 und die Leitfähigkeitsmeßzelle 20 in Figur 2. Um hier die Bewertung der Entkalkung vorzunehmen, ist gegebenenfalls eine Konzentratmischung derart einzustellen, dass eine übersättigte Lösung vorliegt. Sodann wird mittels der stromabwärts befindlichen Leitfähigkeitsmeßzelle die Messung der Leitfähigkeit durchgeführt und sodann die Entkalkungsprozedur vorgenommen. Anschließend wird erneut die genannte Konzentratmischung hergestellt, die Stabilisierung der Leitfähigkeit abgewartet und der Leitfähigkeitsmeßwert mittels der stromabwärtigen Meßzelle erneut gemessen. Eine Entkalkung war zumindest dann teilweise erfolgreich, wenn die Leitfähigkeit nach der Entkalkung größer als die vor der Verkalkung ist. Mittels der oben genannten Gleichung kann auf den Grad der Beseitigung der Verkalkung geschlossen werden. Dieses Verfahren kann

so oft wiederholt werden, bis sich die Leitfähigkeit vor und nach der Entkalkung nicht mehr ändert.

Auch ist es möglich, während der Entkalkung deren Fortschritt zu bewerten. Diese Vorgehensweise beruht auf der Tatsache, dass die spezifische Leitfähigkeit von $H^+$-Ionen diejenige der übrigen in der Dialysierflüssigkeit bzw. in der zur Entkalkung verwendeten Säure um das vier- bis fünffache übersteigt. Bei der Entkalkung eines Filters durch Säure geht $CaCO_3$ unter Verbrauch von $H^+$-Ionen in Lösung, wie dies durch die nachstehend angegebene Reaktionsgleichung dargestellt ist:

$$CaCO_3 + H^+ \rightarrow Ca^{2+} + HCO_3^-$$

Beispielsweise mittels einer Anordnung gemäß Figur 6 kann nun durch einen Vergleich der Leitfähigkeit vor und nach dem Filter der Ablauf der Entkalkung überwacht werden: Die saure Lösung vor dem Filter hat wegen der hohen $H^+$-Ionenkonzentration eine höhere Leitfähigkeit als die nach dem Filter, wenn ein Verbrauch von $H^+$-Ionen durch Reaktion mit $CaCO_3$ eingetreten ist. Die Entkalkung ist genau dann beendet, wenn die Leitfähigkeit stromaufwärts und stromabwärts des Filters identisch ist.

Eine Messung der Leitfähigkeit des Dialysats vor und nach der Entkalkung ist bei diesem Verfahren erforderlich. Alternativ dazu kann die Überwachung während der Entkalkung auch durch pH-Sensoren durchgeführt werden. Wie oben angegeben, erfolgt während der Entkalkung ein Verbrauch von $H^+$-Ionen und somit eine Verschiebung des Milieus ins Alkalische. Ändert sich der pH-Wert nicht mehr, ist die Entkalkung abgeschlossen, da dann ein Verbrauch der $H^+$-Ionen durch $CaCO_3$ nicht mehr erfolgt.

## Patentansprüche

1. Dialysemaschine mit wenigstens einem Filter (D, D1, D2) zur Filtration der Dialysierflüssigkeit sowie mit Mitteln zur Feststellung der Verkalkung der Dialysemaschine, **dadurch gekennzeichnet,** dass die Mittel zur Feststellung der Verkalkung der Dialysemaschine einen oder mehrere Sensoren (1, 2, 10, 20, 30, 40) aufweisen, wobei der oder die Sensoren (1, 2, 10, 20, 30, 40) derart ausgeführt und angeordnet sind, dass mit diesen die Ionenkonzentration oder ein für die Ionenkonzentration oder deren Änderung repräsentativer Parameter der Dialysierflüssigkeit, einer zur Entkalkung dienenden Lösung oder einer sonstigen Meßlösung entweder stromabwärts oder stromaufwärts und stromabwärts des wenigstens einen Filters (D, D1, D2) erfaßbar ist, und dass die Mittel zur Feststellung der Verkalkung der Dialysemaschine ferner eine Auswerte- oder Recheneinheit aufweisen, die derart konfiguriert ist, dass sie auf der Grundlage der oder des mittels des oder der Sensoren (1, 2, 10, 20, 30, 40) erfaßten Ionenkonzentration bzw. Parameterwertes die Verkalkung der Dialysemaschine feststellt.

2. Dialysemaschine nach Anspruch 1, **dadurch gekennzeichnet, dass** es sich bei dem oder den Sensoren (1, 2, 10, 20, 30, 40) um Leitfähigkeitssensoren, ionenselektive Elektroden, pH-Elektroden oder nach spektroskopischen Verfahren arbeitende Sensoren handelt.

3. Dialysemaschine nach Anspruch 1 oder 2, **dadurch gekennzeichnet, dass** es sich bei der Ionenkonzentration um die $Ca^{2+}$-Ionenkonzentration oder um die $H^+$-Ionenkonzentration handelt.

4. Dialysemaschine nach einem der vorhergehenden Ansprüche, **dadurch gekennzeichnet, dass** wenigstens einem der Filter (D, D1, D2) zwei Sensoren (1, 2, 10, 20, 30, 40) zugeordnet sind, von denen einer stromaufwärts und einer stromabwärts des Filters (D, D1, D2) angeordnet ist.

5. Dialysemaschine nach einem der vorhergehenden Ansprüche, **dadurch gekennzeichnet, dass** wenigstens einem der Filter (D1) sowohl auf der Primär-als auch auf der Sekundärseite des Filters (D1) stromaufwärts und stromabwärts jeweils ein Sensor (10, 20, 40) zugeordnet ist.

6. Dialysemaschine nach einem der vorhergehenden Ansprüche, **dadurch gekennzeichnet, dass** wenigstens eine absperrbare Bypassleitung (100) vorgesehen ist, die derart angeordnet ist, dass im geöffneten Zustand der Bypassleitung (100) die Dialysierflüssigkeit, die zur Entkalkung dienende Lösung oder die Meßlösung von einem Sensor (1, 10, 20, 30) unter Umgehung wenigstens eines der Filter (D, D1, D2) zu einem anderen Sensor (2, 40) geführt wird.

7. Dialysemaschine nach einem der vorhergehenden Ansprüche, **dadurch gekennzeichnet, dass** pro Filter (D, D1, D2) oder für mehrere Filter (D1, D2) nur ein Sensor (2, 40) vorgesehen ist, der stromabwärts des oder der Filter (D, D1, D2) angeordnet ist.

8. Dialysemaschine nach Anspruch 7, **dadurch gekennzeichnet, dass** wenigstens eine absperrbare Bypassleitung (200) vorgesehen ist, die derart angeordnet ist, dass im geöffneten Zustand die Dialysierflüssigkeit, die zur Entkalkung dienende Lösung oder die Meßlösung unter Umgehung wenigstens eines Filters (D, D1, D2) dem Sensor (2, 40) zugeführt wird.

9. Verfahren zur Feststellung der Verkalkung einer Dialysemaschine, insbesondere einer Dialysemaschine gemäß einem der Ansprüche 1 bis 8, die wenigstens einen Filter (D, D1, D2) zur Filtration der Dialysierflüssigkeit aufweist, **dadurch gekennzeichnet,** dass zur Feststellung der Verkalkung der Dialysemaschine die Ionenkonzentration oder ein für die Ionenkonzentration oder deren Änderung repräsentativer Parameter der Dialysierflüssigkeit, einer zur Entkalkung dienenden Lösung oder einer sonstigen Meßlösung entweder stromabwärts oder stromaufwärts und stromabwärts des Filters (D, D1, D2) gemessen wird und dass auf der Grundlage der oder des gemessenen Ionenkonzentration bzw. Parameterwertes die Verkalkung der Dialysemaschine festgestellt wird.

10. Verfahren nach Anspruch 9, **dadurch gekennzeichnet, dass** es sich bei dem für die Ionenkonzentration repräsentativen Parameter um die Leitfähigkeit, den pH-Wert oder einen mittels einer ionenselektiven Elektrode oder eines spektroskopischen Verfahrens ermittelten Parameter handelt.

11. Verfahren nach Anspruch 9 oder 10, **dadurch gekennzeichnet, dass** wenigstens einem der Filter (D, D1, D2) zwei Sensoren (1, 2, 10, 20, 30, 40) zugeordnet sind, von denen einer stromaufwärts und einer stromabwärts des Filters (D, D1, D2) angeordnet ist und dass durch einen Vergleich der stromaufwärts und stromabwärts gemessenen Ionenkonzentration oder des für die Ionenenkonzentration oder deren Änderung repräsentativen Parameters die Verkalkung des Filters festgestellt wird.

12. Verfahren nach Anspruch 11, **dadurch gekennzeichnet, dass** zu einem ersten Zeitpunkt, vorzugsweise vor oder zu Beginn einer Behandlung, Differenzen in den Meßwerten der Sensoren (1,2, 10, 20, 30, 40) ermittelt werden, und diese Differenzen sodann zu einem zweiten, nach dem ersten Zeitpunkt liegenden Zeitpunkt, insbesondere während der Behandlung, bei der Feststellung der Verkalkung berücksichtigt werden.

13. Verfahren nach einem der Ansprüche 9 bis 12, **dadurch gekennzeichnet, dass** wenigstens eine absperrbare Bypassleitung (100) vorgesehen ist, die derart angeordnet ist, dass im geöffneten Zustand der Bypassleitung (100) die Dialysierflüssigkeit, die zur Entkalkung dienende Lösung oder die Meßlösung von einem Sensor (1, 10, 20, 30) unter Umgehung wenigstens eines der Filter (D, D1, D2) zu einem anderen Sensor (2, 40) geführt wird, und dass zum Zwecke der Ermittlung von Meßwertdifferenzen der Sensoren (1, 2, 10, 20, 30, 40) oder zur Kalibrierung der Sensoren (1, 2, 10, 20, 30, 40) die Bypassleitung (100) geöffnet wird.

14. Verfahren nach einem der Ansprüche 9 bis 13, **dadurch gekennzeichnet, dass** pro Filter (D, D1, D2) oder für mehrere Filter (D1, D2) nur ein Sensor (40) vorgesehen ist, der stromabwärts des oder der Filter (D, D1, D2) angeordnet ist und dass zu einem ersten Zeitpunkt, vorzugsweise vor oder zu Beginn einer Behandlung, mittels des Sensors (2, 40) ein Meßwert ermittelt wird und dass zu einem zweiten, nach dem ersten Zeitpunkt liegenden Zeitpunkt, insbesondere während der Behandlung, überprüft wird, ob eine Meßwertänderung eintritt.

15. Verfahren nach einem der Ansprüche 9 bis 14, **dadurch gekennzeichnet, dass** pro Filter (D, D1, D2) oder für mehrere Filter (D1, D2) nur ein Sensor (2, 40) vorgesehen ist, der stromabwärts des oder der Filter (D, D1, D2) angeordnet ist und dass wenigstens eine absperrbare Bypassleitung (200) vorgesehen ist, die derart angeordnet ist, dass im geöffneten Zustand der Bypassleitung (200) die Dialysierflüssigkeit, die zur Entkalkung dienende Lösung oder eine sonstige Meßlösung unter Umgehung wenigstens eines Filters (D, D1, D2) dem Sensor (2, 40) zugeführt wird, wobei der Meßwert des Sensors bei geöffneter Bypassleitung (200) ermittelt wird und dieser Meßwert mit dem Meßwert verglichen wird, der nach Durchströmung des Filters (D, D1, D2) erhalten wird.

16. Verfahren nach einem der Ansprüche 9 bis 15, **dadurch gekennzeichnet, dass** eine Entkalkung vorgenommen wird und dass nach oder während der Entkalkung eine Bewertung der Entkalkung vorgenommen wird, die auf einem Vergleich von Meßwerten des einen oder der mehreren Sensoren (1, 2, 10, 20, 30, 40) basiert, mittels derer die Ionenkonzentration oder der für die Ionenkonzentration oder deren Änderung repräsentative Parameter stromaufwärts und stromabwärts des Filters (D, D1, D2) erfaßt wird.

17. Verfahren nach einem der Ansprüche 9 bis 16, **dadurch gekennzeichnet, dass** nach der Entkalkung eine Bewertung der Entkalkung vorgenommen wird, die auf einem Vergleich von Meßwerten eines Sensors (2, 40) vor und nach der Entkalkung basiert, wobei der Sensor (2, 40) stromabwärts des oder der Filter (D, D1, D2) angeordnet und

derart ausgeführt ist, dass mit diesem die Ionenkonzentration oder der für die Ionenkonzentration oder deren Änderung repräsentative Parameter erfaßt wird.

**18.** Verfahren nach einem der Ansprüche 9 bis 17, **dadurch gekennzeichnet, dass** es sich bei der Ionenkonzentration um die $Ca^{2+}$-Ionenkonzentration oder um die $H^+$-Ionenkonzentration handelt.

## Claims

**1.** A dialysis machine comprising at least one filter (D, D1, D2) for the filtration of the dialysis liquid and comprising means for determining the calcification of the dialysis machine, **characterized in that** the means for the determining of the calcification of the dialysis machine comprise one or more sensors (1, 2, 10, 20, 30, 40), with the sensor or sensors (1, 2, 10, 20, 30, 40) being configured and arranged such that the ion concentration or a parameter representative of the ion concentration or of its change of the dialysis liquid, of a solution serving the decalcification or of another measuring solution can be detected either downstream or upstream and downstream of the at least one filter (D, D1, D2); and **in that** the means for determining the calcification of the dialysis machine furthermore comprise an evaluation or calculation unit which is configured such that it determines the calcification of the dialysis machine on the basis of the ion concentration or parameter value detected by means of the sensor or sensors (1, 2, 10, 20, 30, 40).

**2.** A dialysis machine in accordance with claim 1, **characterized in that** the sensor or sensors (1, 2, 10, 20, 30, 40) are conductivity sensors, ion-selective electrodes, pH electrodes or sensors working according to spectroscopic processes.

**3.** A dialysis machine in accordance with either of claims 1 or 2, **characterized in that** the ion concentration is the $Ca^{2+}$ ion concentration or the $H^+$ ion concentration.

**4.** A dialysis machine in accordance with any one of the preceding claims, **characterized in that** two sensors (1, 2, 10, 20, 30, 40) are associated with at least one of the filters (D, D1, D2), of which one is arranged upstream and one downstream of the filter (D, D1, D2).

**5.** A dialysis machine in accordance with any one of the preceding claims, **characterized in that** one respective sensor (10, 20, 40) is associated with at least one of the filters (D1) both on the primary side and on the secondary side of the filter (D1) upstream and downstream.

**6.** A dialysis machine in accordance with any one of the preceding claims, **characterized in that** at least one bypass line (100) is provided which can be cut off and which is arranged such that, in the open state of the bypass line (100), the dialysis liquid, the solution serving the decalcification or the measuring solution is guided by a sensor (1, 10, 20, 30) while bypassing at least one of the filters (D, D1, D2) to another sensor (2, 40).

**7.** A dialysis machine in accordance with any one of the preceding claims, **characterized in that** only one sensor (2, 40) is provided per filter (D, D1, D2) or for a plurality of filters (D1, D2) and is arranged downstream of the filter or filters (D, D1, D2).

**8.** A dialysis machine in accordance with claim 7, **characterized in that** at least one bypass line (200) is provided which can be cut off and is arranged such that, in the open state, the dialysis liquid, the solution serving the decalcification or the measuring solution is supplied to the sensor (2, 40) while bypassing at least one filter (D, D1, D2).

**9.** A method of determining the calcification of a dialysis machine, in particular of a dialysis machine in accordance with one of the claims 1 to 8, which comprises at least one filter (D, D1, D2) for the filtration of the dialysis liquid, **characterized in that** the ion concentration or a parameter representative of the ion concentration or of its change of the dialysis liquid, of a solution serving the decalcification or of another measuring solution is measured either downstream or upstream and downstream of the filter (D, D1, D2) for the determining of the calcification of the dialysis machine; and **in that** the calcification of the dialysis machine is determined on the basis of the ion concentration or the parameter value measured.

**10.** A method in accordance with claim 9, **characterized in that** the parameter representative of the ion concentration is the conductivity, the pH or a parameter determined by means of an ion-selective electrode or of a spectroscopic

method.

**11.** A method in accordance with either of claims 9 or 10, **characterized in that** two sensors (1, 2, 10, 20, 30, 40) are associated with at least one of the filters (D, D1, D2), one of which is arranged upstream and one downstream of the filter (D, D1, D2); and **in that** the calcification of the filter is determined by a comparison of the ion concentration or of the parameter representative of the ion concentration or of its change measured upstream and downstream.

**12.** A method in accordance with claim 11, **characterized in that** differences in the measured values of the sensors (1, 2, 10, 20, 30, 40) are determined at a first point in time, preferably before or at the start of a treatment, and these differences are then taken into account in the determination of the calcification at a second point in time disposed after the first time point, in particular during the treatment.

**13.** A method in accordance with any one of the claims 9 to 12, **characterized in that** at least one bypass line (100) is provided which can be cut off and which is arranged such that, in the open state of the bypass line (100), the dialysis liquid, the solution serving the decalcification or the measuring solution is guided from a sensor (1, 10, 20, 30) to another sensor (2, 40) while bypassing at least one of the filters (D, D1, D2); and **in that** the bypass line (100) is opened for the purpose of determining measured value differences of the sensors (1, 2, 10, 20, 30, 40) or for the calibration of the sensors (1, 2, 10, 20, 30, 40).

**14.** A method in accordance with any one of the claims 9 to 13, **characterized in that** only one sensor (40) is provided per filter (D, D1, D2) or for a plurality of filters (D1, D2) and is arranged downstream of the filter or filters (D, D1, D2); and **in that** a measured value is determined by means of the sensor (2, 40) at a first point in time, preferably before or at the start of a treatment; and **in that** a check is made after a second point in time disposed after the first point in time, in particular during the treatment, whether a measured value change occurs.

**15.** A method in accordance with any one of the claims 9 to 14, **characterized in that** only one sensor (2, 40) is provided per filter (D, D1, D2) or for a plurality of filters (D1, D2) and is arranged downstream of the filter or filters (D, D1, D2); and **in that** at least one bypass line (200) is provided which can be cut off and which is arranged such that, in the open state of the bypass line (200), the dialysis liquid, the solution serving the decalcification or another measuring solution is supplied to the sensor (2, 40) while bypassing at least one filter (D, D1, D2), with the measured value of the sensor being determined with an open bypass line (200) and this measured value being compared with the measured value obtained after flowing through the filter (D, D1, D2).

**16.** A method in accordance with any one of the claims 9 to 15, **characterized in that** a decalcification is carried out; and **in that** an evaluation of the decalcification is made after the decalcification or during the decalcification which is based on a comparison of measured values of the one or several sensors (1, 2, 10, 20, 30, 40) by means of which the ion concentration or the parameter representative of the ion concentration or of its change is detected upstream and downstream of the filter (D, D1, D2).

**17.** A method in accordance with any one of the claims 9 to 16, **characterized in that** an evaluation of the decalcification is carried out after the decalcification which is based on a comparison of measured values of a sensor (2, 40) before and after the decalcification, with the sensor (2, 40) being arranged downstream of the filter or filters (D, D1, D2) and being configured such that the ion concentration or the parameter representative of the ion concentration or of its change is detected.

**18.** A method in accordance with any one of the claims 9 to 17, **characterized in that** the ion concentration is the $Ca^{2+}$ ion concentration or the $H^+$ ion concentration.

## Revendications

**1.** Machine à dialyse comprenant au moins un filtre (D, D1, D2) destiné à la filtration du fluide dialyseur ainsi que des moyens de détermination de l'entartrage de la machine à dialyse, **caractérisée en ce que** les moyens de détermination de l'entartrage de la machine à dialyse comportent un ou plusieurs capteurs (1, 2, 10, 20, 30, 40), le ou les capteurs (1, 2, 10, 20, 30, 40) étant réalisés et disposés de telle manière qu'avec ceux-ci, la concentration en ions ou un paramètre représentatif de la concentration en ions ou de sa modification du fluide dialyseur, d'une solution servant au détartrage ou d'une autre solution de mesure puisse être détecté(e) soit en aval soit en amont et en aval de l'au moins un filtre (D, D1, D2), et **en ce que** les moyens de détermination de l'entartrage de la machine à dialyse

comportent en outre une unité d'évaluation ou de calcul qui est configurée de telle manière qu'elle détermine l'entartrage de la machine à dialyse sur la base de la concentration en ions ou valeur de paramètre détectée au moyen du ou des capteurs (1,2, 10, 20, 30, 40).

**2.** Machine à dialyse selon la revendication 1, **caractérisée en ce que** le ou les capteurs (1, 2, 10, 20, 30, 40) sont des capteurs de conductivité, des électrodes sélectives d'ions, des électrodes de mesure du pH ou des capteurs fonctionnant selon un procédé spectroscopique.

**3.** Machine à dialyse selon la revendication 1 ou 2, **caractérisée en ce que** la concentration en ions est la concentration en ions $Ca^{2+}$ ou la concentration en ions $H^{+}$.

**4.** Machine à dialyse selon l'une des revendications précédentes, **caractérisée en ce que** deux capteurs (1, 2, 10, 20, 30, 40) sont associés à au moins l'un des filtres (D, D1, D2), l'un des capteurs étant disposé en amont et l'autre en aval du filtre (D, D1, D2).

**5.** Machine à dialyse selon l'une des revendications précédentes, **caractérisée en ce qu'**un capteur (10, 20, 40) est associé à au moins l'un des filtre (D1) respectivement en amont et en aval du côté primaire ainsi que du côté secondaire du filtre (D1).

**6.** Machine à dialyse selon l'une des revendications précédentes, **caractérisée en ce qu'**au moins une ligne de dérivation (100) pouvant être fermée est prévue, disposée de telle manière qu'à l'état ouvert de la ligne de dérivation (100) le fluide dialyseur, la solution servant au détartrage ou la solution de mesure est conduit(e) d'un capteur (1, 10, 20, 30) à un autre capteur (2, 40) en contournant au moins l'un des filtres (D, D1, D2).

**7.** Machine à dialyse selon l'une des revendications précédentes, **caractérisée en ce qu'**un seul capteur (2, 40) est prévu par filtre (D, D1, D2) ou pour plusieurs filtres (D1, D2), le capteur étant disposé en aval du ou des filtres (D, D1, D2).

**8.** Machine à dialyse selon la revendication 7, **caractérisée en ce qu'**au moins une ligne de dérivation (200) pouvant être fermée est prévue, disposée de telle manière qu'à l'état ouvert le fluide dialyseur, la solution servant au détartrage ou la solution de mesure est acheminé(e) au capteur (2, 40) en contournant au moins un filtre (D, D1, D2).

**9.** Procédé de détermination de l'entartrage d'une machine à dialyse, en particulier d'une machine à dialyse selon l'une des revendications 1 à 8, qui comporte au moins un filtre (D, D1, D2) destiné à la filtration du fluide dialyseur, **caractérisé en ce que** pour déterminer l'entartrage de la machine à dialyse la concentration en ions ou un paramètre représentatif de la concentration en ions ou de sa modification du fluide dialyseur, d'une solution servant au détartrage ou d'une autre solution de mesure est mesuré(e) soit en aval soit en amont et en aval du filtre (D, D1, D2) et **en ce que** l'entartrage de la machine à dialyse est déterminé sur la base de la concentration en ions ou valeur de paramètre mesurée.

**10.** Procédé selon la revendication 9, **caractérisé en ce que** le paramètre représentatif de la concentration en ions est la conductivité, la valeur de pH ou un paramètre déterminé au moyen d'une électrode sélective d'ions ou d'un procédé spectroscopique.

**11.** Procédé selon la revendication 9 ou 10, **caractérisé en ce que** deux capteurs (1, 2, 10, 20, 30, 40) sont associés à au moins l'un des filtres (D, D1, D2), l'un des capteurs étant disposé en amont et l'autre en aval du filtre (D, D1, D2) et **en ce que** l'entartrage du filtre est déterminé par une comparaison de la concentration en ions ou du paramètre représentatif de la concentration en ions ou de sa modification mesuré(e) en amont et en aval.

**12.** Procédé selon la revendication 11, **caractérisé en ce que** des différences dans les valeurs mesurées des capteurs (1, 2, 10, 20, 30, 40) sont déterminées à un premier instant, de préférence avant ou au début d'un traitement, et ces différences sont ensuite prises en compte à un second instant situé après le premier instant, en particulier pendant le traitement, lors de la détermination de l'entartrage.

**13.** Procédé selon l'une des revendications 9 à 12, **caractérisé en ce qu'**au moins une ligne de dérivation (100) pouvant être fermée est prévue, disposée de telle manière qu'à l'état ouvert de la ligne de dérivation (100) le fluide dialyseur, la solution servant au détartrage ou la solution de mesure est conduit(e) d'un capteur (1, 10, 20, 30) à un autre capteur (2, 40) en contournant au moins l'un des filtres (D, D1, D2), et **en ce que** la ligne de dérivation (100) est

ouverte dans le but de déterminer des différences de valeurs mesurées des capteurs (1, 2, 10, 20, 30, 40) ou pour l'étalonnage des capteurs (1, 2, 10, 20, 30, 40).

**14.** Procédé selon l'une des revendications 9 à 13, **caractérisé en ce qu'**un seul capteur (40) est prévu par filtre (D, D1, D2) ou pour plusieurs filtres (D1, D2), le capteur étant disposé en aval du ou des filtres (D, D1, D2) et **en ce qu'**à un premier instant, de préférence avant ou au début d'un traitement, une valeur de mesure est déterminée au moyen du capteur (2, 40) et **en ce qu'**à un second instant situé après le premier instant, en particulier pendant le traitement, on vérifie si une modification de la valeur mesurée se produit.

**15.** Procédé selon l'une des revendications 9 à 14, **caractérisé en ce qu'**un seul capteur (2,40) est prévu par filtre (D, D1, D2) ou pour plusieurs filtres (D1, D2), le capteur étant disposé en aval du ou des filtres (D, D1, D2) et **en ce qu'**au moins une ligne de dérivation (200) pouvant être fermée est prévue, disposée de telle manière qu'à l'état ouvert de la ligne de dérivation (200) le fluide dialyseur, la solution servant au détartrage ou une autre solution de mesure est acheminé(e) au capteur (2, 40) en contournant au moins un filtre (D, D1, D2), la valeur mesurée du capteur lorsque la ligne de dérivation (200) est ouverte étant déterminée et cette valeur mesurée étant comparée à la valeur mesurée qui est obtenue après la traversée du filtre (D, D1, D2).

**16.** Procédé selon l'une des revendications 9 à 15, **caractérisé en ce qu'**un détartrage est effectué et **en ce qu'**après ou pendant le détartrage est effectuée une évaluation du détartrage qui est basée sur une comparaison de valeurs mesurées du ou des plusieurs capteurs (1, 2, 10, 20, 30, 40), au moyen de laquelle la concentration en ions ou le paramètre représentatif de la concentration en ions ou de sa modification est détecté(e) en amont et en aval du filtre (D, D1, D2).

**17.** Procédé selon l'une des revendications 9 à 16, **caractérisé en ce qu'**une évaluation du détartrage est effectuée après le détartrage, qui est basée sur une comparaison de valeurs mesurées d'un capteur (2, 40) avant et après le détartrage, le capteur (2,40) étant disposé en aval du ou des filtres (D, D1, D2) et réalisé de telle manière qu'avec celui-ci la concentration en ions ou le paramètre représentatif de la concentration en ions ou de sa modification est détecté(e).

**18.** Procédé selon l'une des revendications 9 à 17, **caractérisé en ce que** la concentration en ions est la concentration en ions $Ca^{2+}$ ou la concentration en ions $H^+$.

# Figur 1

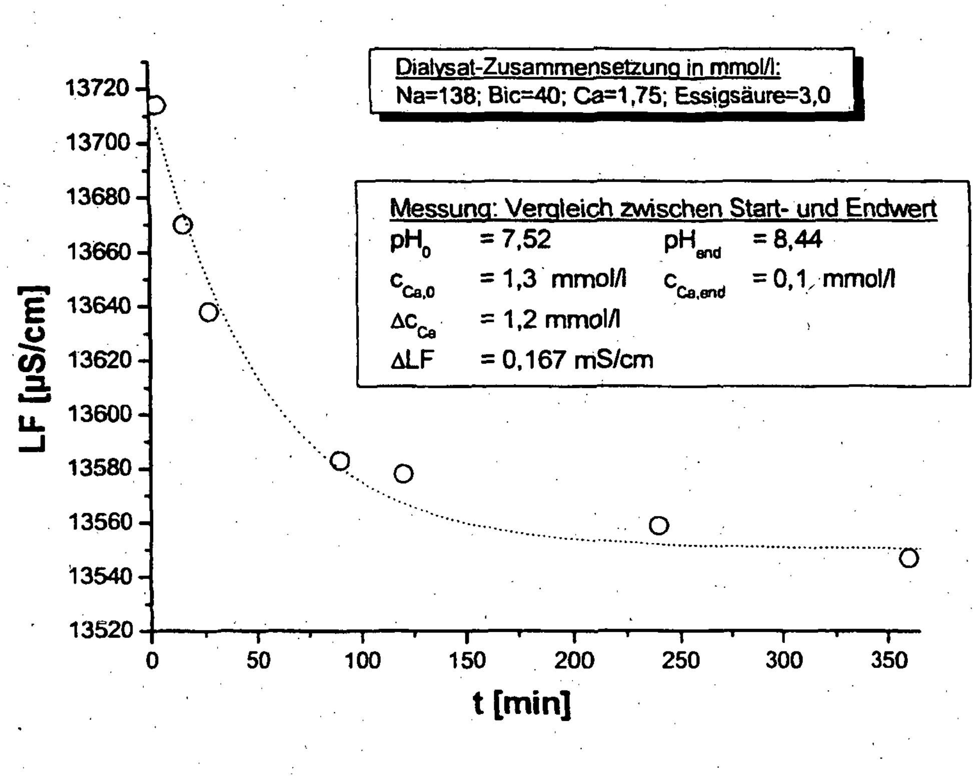
Dialysat-Zusammensetzung in mmol/l:
Na=138; Bic=40; Ca=1,75; Essigsäure=3,0
Messung: Vergleich zwischen Start- und Endwert
pH0 = 7,52
pHend = 8,44
cCa,0 = 1,3 mmol/l
cCa,end = 0,1 mmol/l
ΔcCa = 1,2 mmol/l
ΔLF = 0,167 mS/cm
LF [µS/cm]
13720
13700
13680
13660
13640
13620
13600
13580
13560
13540
13520
0
50
100
150
200
250
300
350
t [min]

# Figur 2

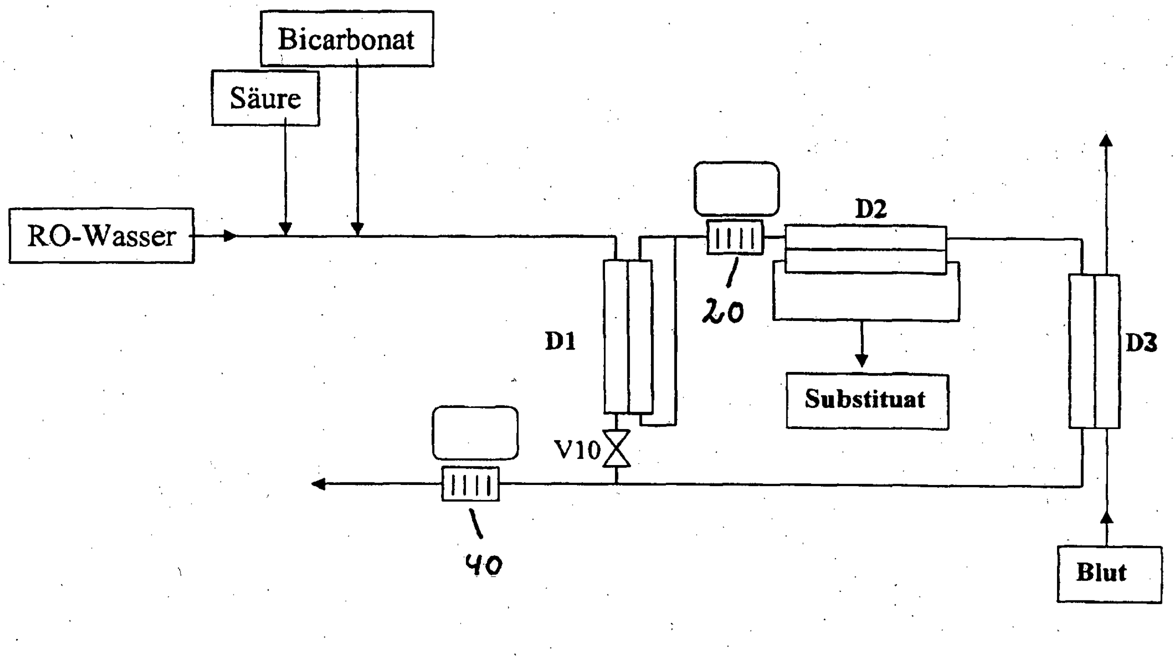
Bicarbonat
Säure
RO-Wasser
D2
20
D1
D3
Substituat
V10
40
Blut

# Figur 3

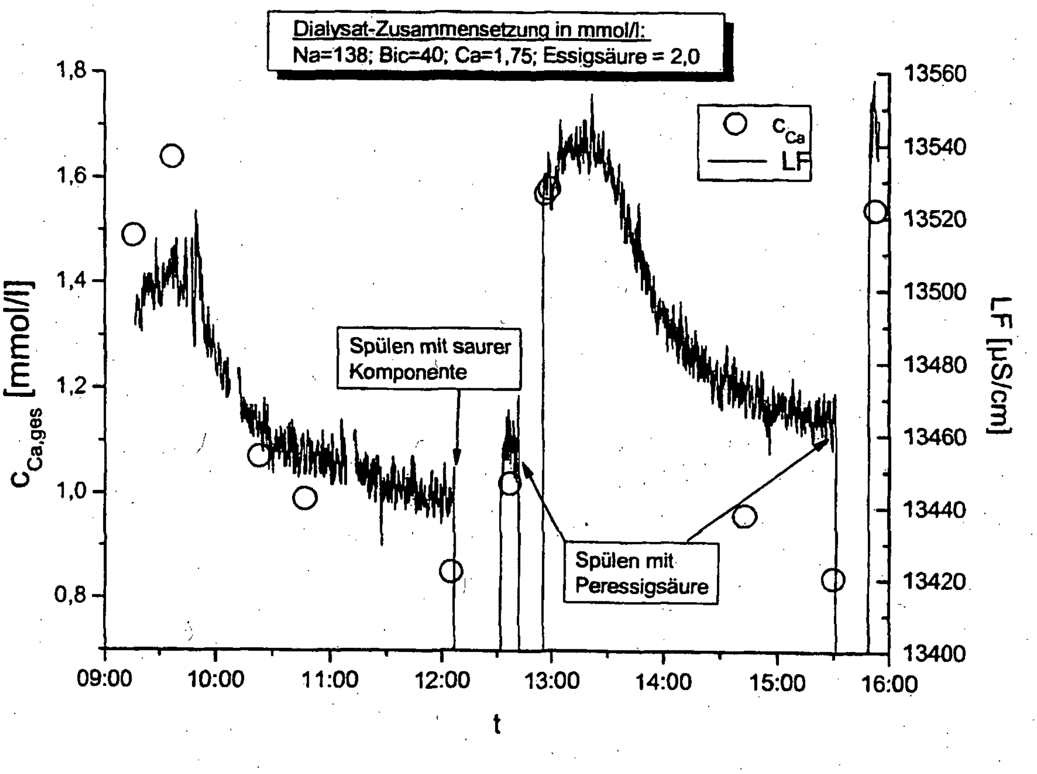
Dialysat-Zusammensetzung in mmol/l:
Na=138; Bic=40; Ca=1,75; Essigsäure = 2,0
1,8
1,6
1,4
1,2
1,0
0,8
$c_{Ca,ges}$ [mmol/l]
13560
13540
13520
13500
13480
13460
13440
13420
13400
LF [µS/cm]
$c_{Ca}$
LF
Spülen mit saurer Komponente
Spülen mit Peressigsäure
09:00
10:00
11:00
12:00
13:00
14:00
15:00
16:00
t

# Figur 4

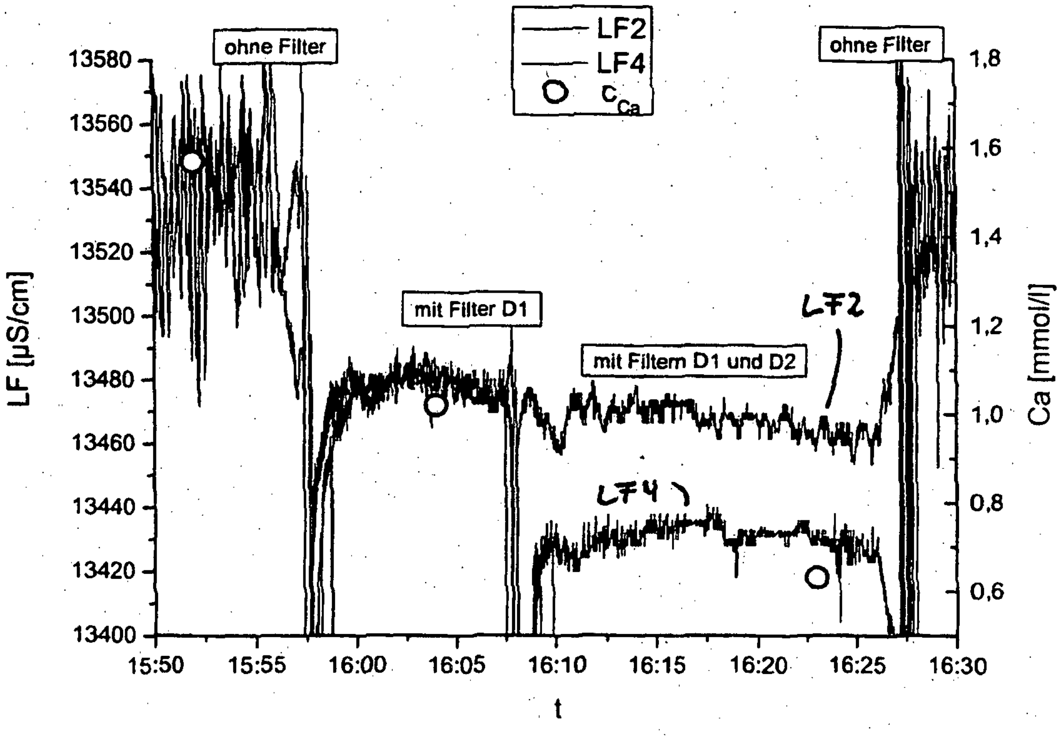
LF2
LF4
$c_{Ca}$
ohne Filter
mit Filter D1
mit Filtern D1 und D2
ohne Filter
LF2
LF4
LF [µS/cm]
13580
13560
13540
13520
13500
13480
13460
13440
13420
13400
Ca [mmol/l]
1,8
1,6
1,4
1,2
1,0
0,8
0,6
15:50
15:55
16:00
16:05
16:10
16:15
16:20
16:25
16:30
t

Figur 5

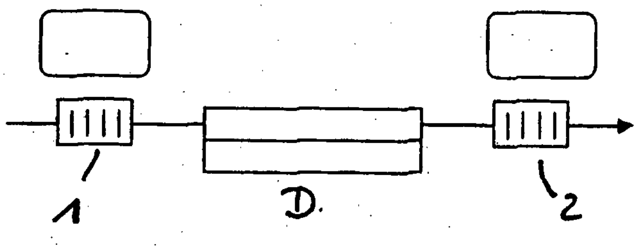


Figur 6

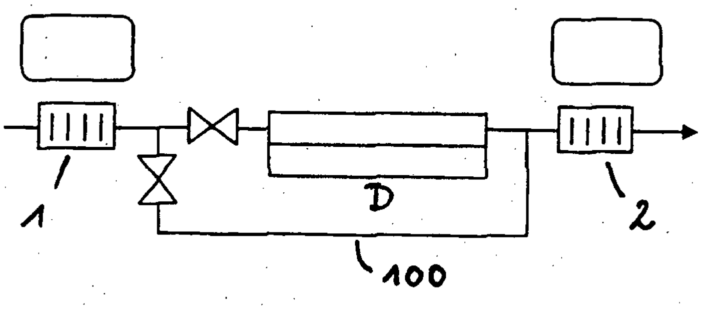


Figur 7

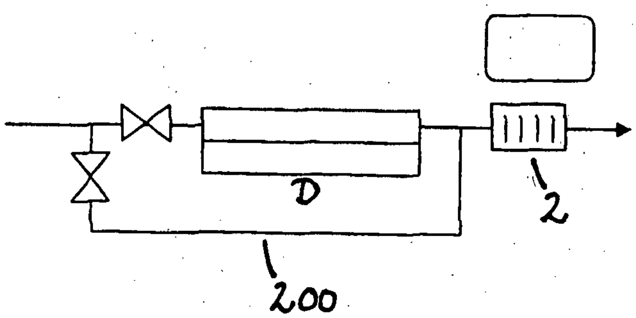

Figur 8

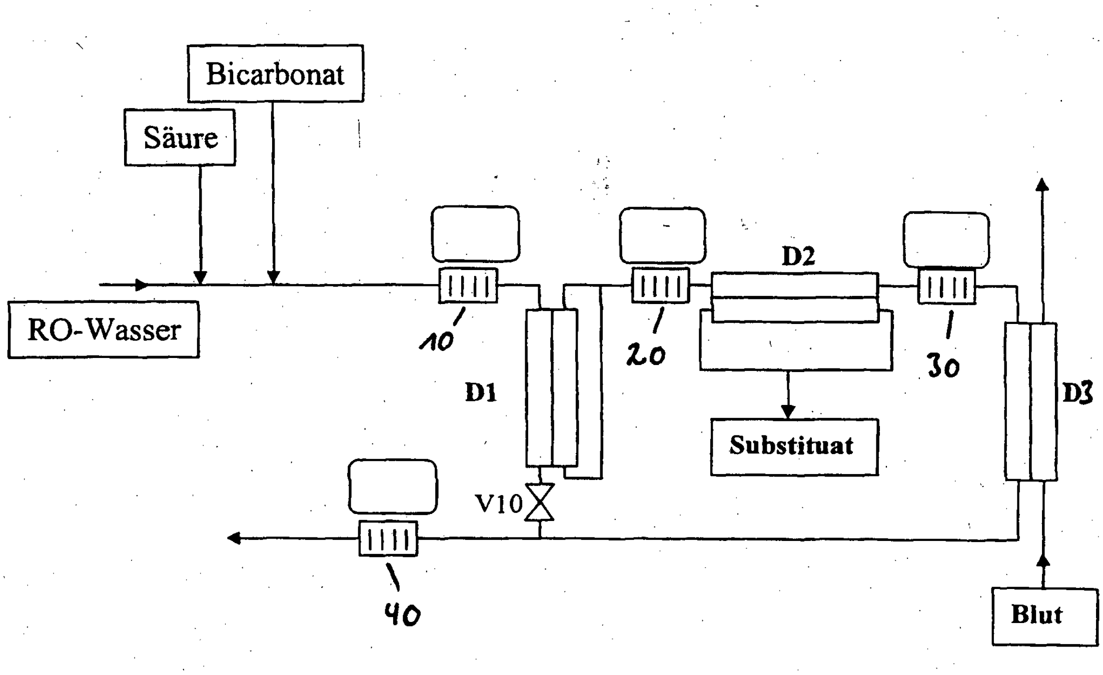
Bicarbonat
Säure
RO-Wasser
10
D1
V10
20
D2
Substituat
30
D3
40
Blut

Figur 9

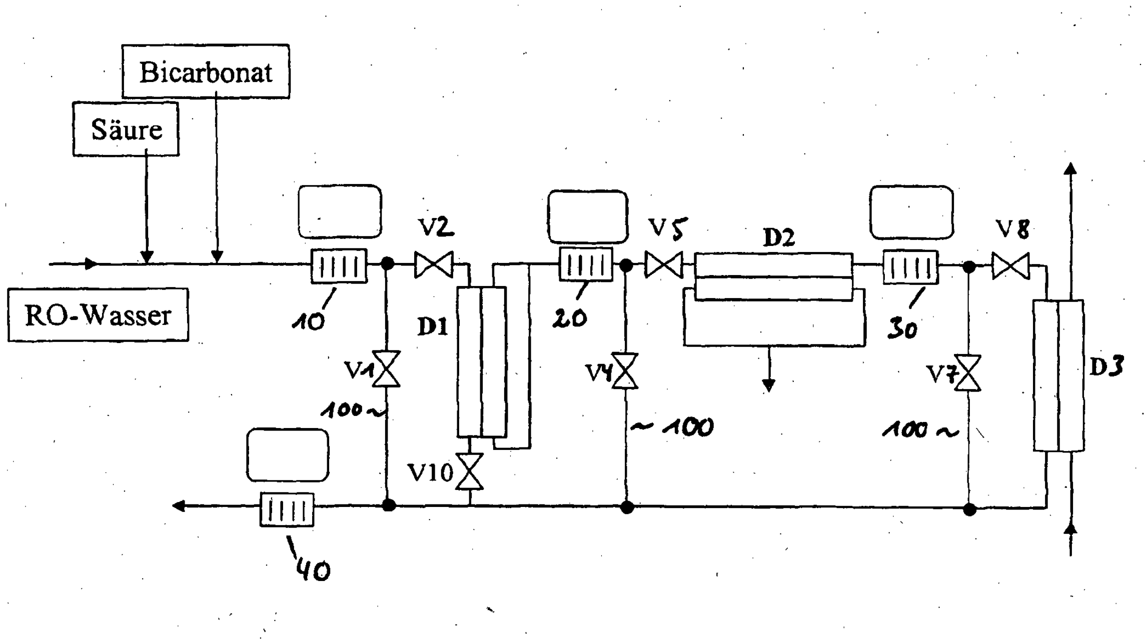
Bicarbonat
Säure
RO-Wasser
10
V2
D1
20
V5
D2
30
V8
D3
V1
100
V4
100
V7
100
V10
40

# Figur 10

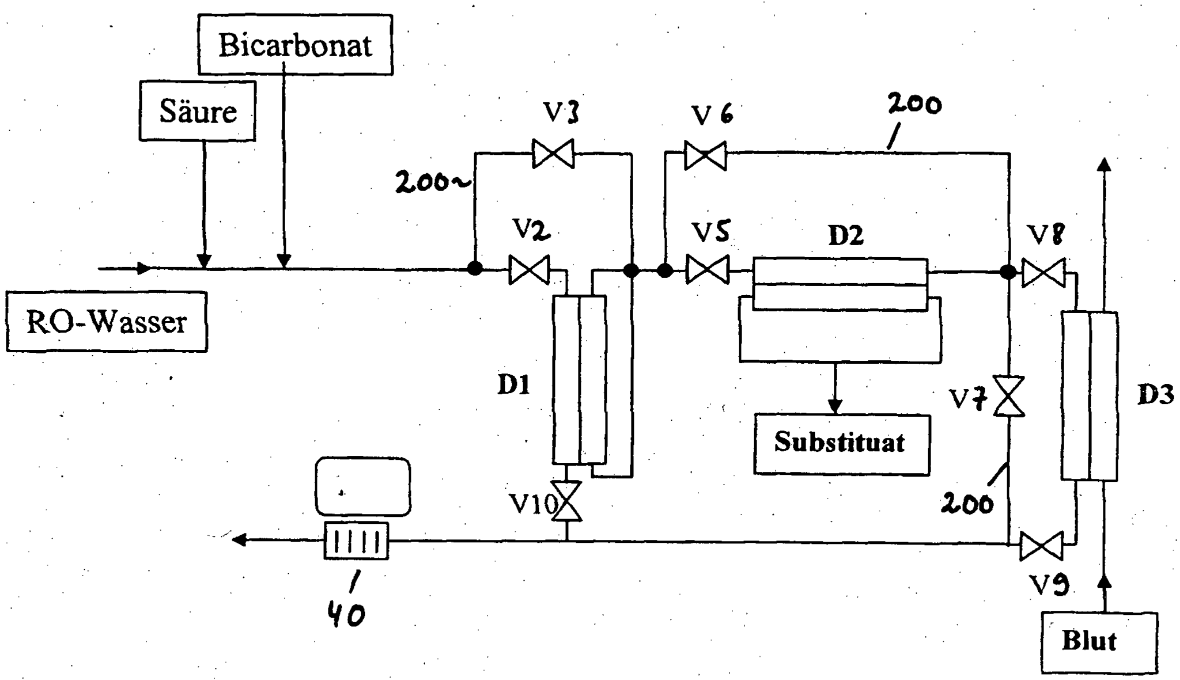
Bicarbonat
Säure
V3
V 6
200
200
V2
V5
D2
V8
RO-Wasser
D1
V7
D3
Substituat
V10
200
40
V9
Blut

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0834328 A1